# EUROPEAN PATENT APPLICATION

(11) **EP 1 998 169 A1**
(43) Date of publication of application: **03.12.2008**
(21) Application number: 07714876.5
(22) Date of filing: 23.02.2007
(51) Int. Cl.: G01N 21/77, G01N 31/00, G01N 31/22, G01N 21/47

(54) **HYDROGEN SENSOR**

(30) Priority: 20.03.2006 JP 2006076352
(71) Applicant: KABUSHIKI KAISHA ATSUMITEC, Hamamatsu-shi Shizuoka 431-0192 (JP)
(72) Inventor: UCHIYAMA, Naoki, Hamamatsu-shi, Shizuoka 431-0192 (JP); MATSUMOTO, Hiroyuki, Hamamatsu-shi, Shizuoka 431-0192 (JP); NAKABAYASHI, Seigou, Hamamatsu-shi, Shizuoka 431-0192 (JP)
(74) Representative: Burt, Matthew Thomas
(86) International application number: PCT/JP2007/053421
(87) International publication number: WO 2007/108276

(57) **Abstract**

A hydrogen sensor includes a thin film layer formed over a substrate of resin or the like, and a catalyst layer formed on a surface of the thin film layer. When contacted by leaked hydrogen gas, the catalyst layer quickly hydrogenates the thin film layer through its catalytic action, thereby causing a change in optical reflectance of the thin film layer. The hydrogen sensor includes a protective film formed at least either between the substrate and the thin film layer or on a surface of the catalyst layer.

## Description

### Technical Field

This invention relates to a hydrogen sensor for detecting hydrogen gas in an atmosphere.

### Background Art

Hydrogen has been attracting attention as an energy source which leads to reduction of carbon dioxide emissions. There is a possibility, however, that if hydrogen gas leaks into an atmosphere (atmosphere in an underground parking lot for hydrogen fuel cell vehicles, at a hydrogen gas station, etc., for example), the leaked hydrogen gas may explode. Thus, it is necessary to quickly detect a hydrogen gas leak and stop it. A semiconductor sensor using tin oxide is used as a hydrogen sensor for detecting such leaked hydrogen gas.

This semiconductor sensor can, however, not detect hydrogen gas unless heated to 400°C or so. Such heating requires a measure for preventing explosion of leaked hydrogen gas, which results in complicated structure, and therefore, high cost of the device for detecting leaked hydrogen gas.

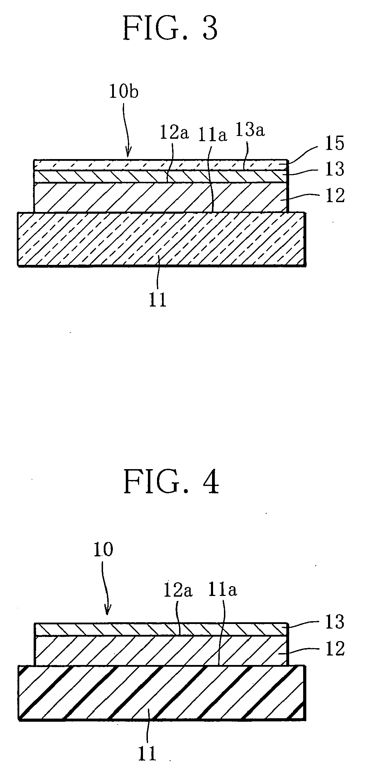

In this connection, a hydrogen sensor as shown in FIG. 4, which does not require heating, therefore, does not require an explosion prevention measure, is proposed in Unexamined Japanese Patent Publication No. 2005-83832 (hereinafter referred to as Patent Document 1). As shown in FIG. 4, this hydrogen sensor 10 includes a thin film layer 12 formed on a surface 11a of a substrate 11 of resin, glass or the like (vinyl sheet, for example), and a catalyst layer 13 formed on a surface 12a of the thin film layer 12. When contacted by leaked hydrogen gas, the catalyst layer 13 quickly hydrogenates the thin film layer 12 through its catalytic action, thereby causing a change in optical reflectance of the thin film layer 12.

In the hydrogen sensor 10 having a thin film layer 12 formed on a surface 11a of a substrate 11 of a hygroscopic substance such as resin, however, it is unavoidable that water, oxygen and/or the like, contained in the substrate 11 or absorbed into the substrate 11 from the atmosphere, penetrates into the thin film layer 12, oxidizes and thereby deteriorates the thin film layer 12. The thin film layer 12 deteriorated in this manner may not allow quick detection of leaked hydrogen gas. Even when a substrate 11 of glass, which is less hygroscopic, is used, the thin film layer 12 can deteriorate, since glass is not non-hygroscopic. Further, by absorbing water, oxygen and/or the like from the atmosphere, the catalytic layer 13 deteriorates, namely its catalytic ability declines, and if the absorbed water, oxygen and/or the like penetrates further into the thin film layer 12, the thin film layer 12 deteriorates. The catalytic layer 13 or thin film layer 12 deteriorated in this manner may not allow quick detection of leaked hydrogen gas.

### Disclosure of the Invention

The present invention is applied to a hydrogen sensor comprising a thin film layer formed over a substrate of glass, resin or the like, and a catalytic layer formed on a surface of the thin film layer, intended such that when contacted by leaked hydrogen gas, the catalyst layer quickly hydrogenates the thin film layer through its catalytic action, thereby causing a change in optical reflectance of the thin film layer.

In order to solve the problems as mentioned above, an object of the present invention is to provide a hydrogen sensor capable of preventing water, oxygen and/or the like, contained in the substrate or absorbed into the substrate from the atmosphere, from penetrating into the thin film layer, thereby preventing deterioration of the catalyst layer or the thin film layer.

In order to solve the problems as mentioned above, another object of the present invention is to provide a hydrogen sensor capable of preventing the catalyst layer from absorbing water, oxygen and/or the like from the atmosphere, thereby preventing deterioration of the catalyst layer or the thin film layer.

In order to achieve the above objects, a hydrogen sensor according to the present invention comprises: a substrate; a thin film layer formed over the substrate; and a catalyst layer formed on a surface of the thin film layer for causing the thin film layer to be hydrogenated by hydrogen gas in an atmosphere, thereby causing a change in optical reflectance of the thin film layer, wherein a protective film is formed at least either between the substrate and the thin film layer or on a surface of the catalyst layer.

The protective film formed between the substrate and the thin film layer of the hydrogen sensor can prevent the water, oxygen and/or the like absorbed into the substrate from penetrating into the protective film. The protective film formed on the surface of the catalyst layer can prevent the catalyst layer from absorbing water, oxygen and/or the like from the atmosphere. Consequently, deterioration of the protective film and the catalyst layer can be prevented.

Since the hydrogen sensor according to the present invention can prevent deterioration of the thin film layer and the catalyst layer as mentioned above, its hydrogen-gas detection function, namely the function of detecting leaked hydrogen gas can be maintained satisfactorily for a long period of time.

Specifically, the thin film layer may be a magnesium-nickel alloy thin film layer or a magnesium thin film layer.

The magnesium-nickel alloy thin film layer or the magnesium thin film layer provided as the aforementioned thin film layer of the hydrogen sensor can exhibit a change in optical reflectance when contacted by leaked hydrogen gas.

Specifically, the catalyst layer may be formed of palladium or platinum.

The catalyst layer formed of palladium or platinum can hydrogenate the thin film layer through its catalytic action.

More specifically, the catalyst layer may have a thickness between 1nm and 100nm.

The catalyst layer with a thickness between 1nm and 100nm can quickly hydrogenate the thin film layer, when contacted by leaked hydrogen gas.

Specifically, the protective film may be formed of a silicon compound, a fluorine compound, a fat or an oil.

The protective film formed of a silicon compound, a fluorine compound, a fat or an oil (such as a mineral oil or a vegetable oil) can prevent water, oxygen and/or the like in the atmosphere from penetrating into the thin film layer and the catalyst layer, and, at the same time, it can allow the catalyst layer to act as a catalyst.

More specifically, the protective film may have a thickness between 5nm and 200nm.

The protective film with a thickness between 5nm and 200nm hardly obstructs the catalyst layer in quickly hydrogenating the thin film.

### Brief Description of the Drawings

FIG. 1 illustrates, in section, an exemplary structure of a hydrogen sensor according to one embodiment of the present invention;
FIG. 2 is a graph showing deterioration (temporal deterioration) of a thin film layer with a protective film and of a thin film layer without a protective film;
FIG. 3 illustrates, in section, an exemplary structure of a modification of the hydrogen sensor shown in FIG. 1; and
FIG. 4 illustrates an exemplary structure of a conventional hydrogen sensor.

### Best Mode of Carrying out the Invention

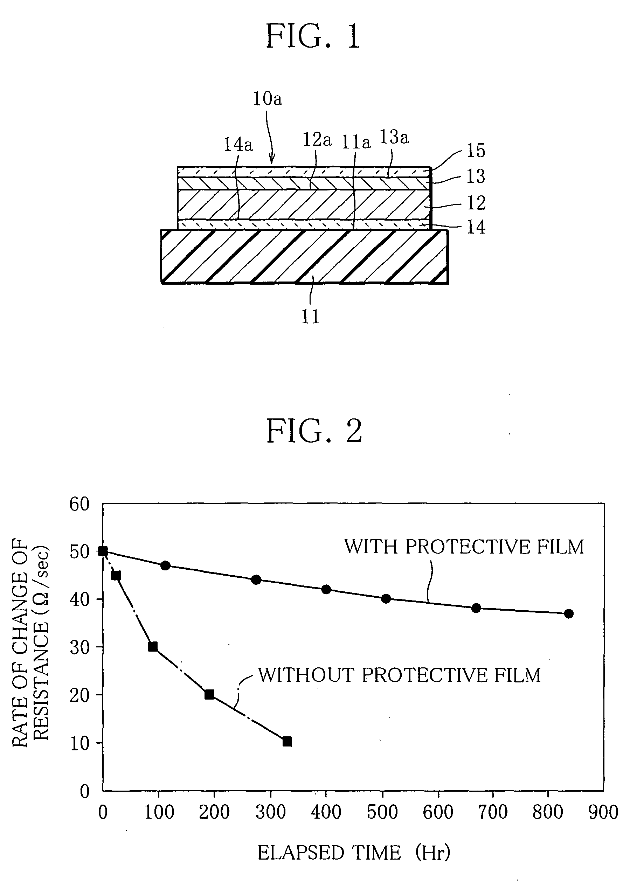

Referring to FIG. 1, a hydrogen sensor according to one embodiment of the present invention will be described below, where the constituents similar in function to those of the conventional hydrogen sensor 10 will be assigned the same reference signs, and the description of such constituents will be omitted.

The hydrogen sensor 10a shown in FIG. 1 includes a first protective film 14 of silicon dioxide (SiO₂) formed on a surface 11a of a substrate 11 consisting of an acrylic resin, a polyethylene sheet (polyethylene film) or the like, and a thin film layer 12 of magnesium-nickel alloy or magnesium formed on a surface 14a of the first protective film 14. Further, a catalyst layer 13 of palladium or platinum is formed on a surface 12a of the thin film layer 12, and a second protective film 15 of silicon dioxide is formed on a surface 13a of the catalyst layer 13.

Thus, the hydrogen sensor 10a has a first protective film 14 between the surface 11a of the substrate 11 and the thin film layer 12, and a second protective film 15 on the surface 13a of the catalyst layer 13. In the hydrogen sensor 10a with this structure, the first protective film 14 prevents the water, oxygen and/or the like contained in the substrate 11 due to absorption or the like from penetrating into the thin film layer 12, and the second protective film 15 prevents the water, oxygen and/or the like contained in the atmosphere from being absorbed into the catalyst layer 13, so that deterioration of the thin film layer 12 and the catalyst layer 13 is prevented.

FIG. 2 is a graph showing deterioration (temporal deterioration) of the thin film layer 12 and catalyst layer 13 with a protective film and of those without a protective film. When hydrogenated by hydrogen contacting it, the thin film layer 12 changes in optical reflectance and increases in electric resistance. The thin film layer 12 also increases in electric resistance when oxidized (deteriorated). Thus, the deterioration of the thin film layer 12 can be evaluated from the increase in electric resistance, as follows:

On a coordinate system for resistance value versus time, a point representing a resistance value measured immediately after the structure including the thin film layer 12 and catalyst layer 13 over the substrate 11 is formed, without letting hydrogen gas contact the formed structure, and a point representing a resistance value measured a predetermined time (about 10 seconds) after the formed structure is brought in contact with hydrogen gas are plotted, and the gradient of a line connecting the two points, namely a change of resistance value per second is obtained. After this, at intervals of a predetermined time, the change of resistance value per second of the formed structure is repeatedly obtained in the same manner as described above. If the change of resistance value per second has decreased little with time, it can be considered that the thin film layer 12 has deteriorated little. If, on the other hand, the change of resistance value per second has decreased in a certain degree with time, it can be considered that the thin film layer 12 has deteriorated. Thus, FIG. 2 demonstrates that in the hydrogen sensor with a protective film, the thin film layer deteriorates little, while in the hydrogen sensor without a protective film, the thin film layer deteriorates.

The thin film layer 12 can be formed by sputtering, vacuum evaporation, electron-beam evaporation, plating or the like. The composition of the thin film layer 12 is MgNix (0≤x<0.6), for example. The catalyst layer 13 can be formed on the surface 12a of the thin film layer 12 by coating or the like. The thickness of the catalyst layer 13 is 1nm to 100nm. The first protective film 14 can be formed by sputtering, vacuum evaporation, plating or the like. The second protective film 15 can be formed by spraying, dip coating, spin coating, brush coating, or any of the processes mentioned for the first protective film 14.

It is to be noted that the first protective film 14 needs to be formed with a smooth surface 14a to allow the thin film layer 12 to be formed on the surface 14a, while the surface of the second protective film 15 does not need to be so smooth as the surface 14a of the protective film 14.

The thickness of the first protective film 14 may be determined appropriately, in accordance with the hygroscopicity of the substrate 11 (degree to which the substrate absorbs water, oxygen and/or the like), etc. Preferably, the thickness of the second protective film 15 is within the range of 5nm to 200nm. The second protective film 15 with such thickness can prevent the catalyst layer 13 from absorbing water, oxygen, and/or the like from the atmosphere, and, at the same time, it can allow the catalyst layer 13 to quickly act as a catalyst (i.e., allow the catalyst layer 13 to quickly hydrogenate the thin film layer 12, when contacted by hydrogen gas in the atmosphere).

Thus, in the hydrogen sensor 10a, when contacted by the atmosphere with a hydrogen concentration no less than the lower limit set within the range of 100ppm to 1%, the catalyst layer 13 can hydrogenate the thin film layer 12 through its catalytic action, thereby causing a rapid change in optical reflectance of the thin film layer 12 in several to 10 seconds or so. Consequently, the hydrogen sensor 10a can quickly detect a hydrogen gas leak.

Even with the second protective film 15 with a thickness below the above-mentioned range, the hydrogen sensor 10a can reduce the amount of water, oxygen, and/or the like penetrating from the atmosphere into the catalyst layer 13 and the thin film layer 12, thereby reducing the degree of deterioration of the catalyst layer 13 and the thin film layer 12 and maintaining its hydrogen-gas detection function satisfactorily for a relatively long period of time. On the other hand, the second protective film 15 with a thickness exceeding the above-mentioned range leads to an increase in time taken for the catalyst layer 13 to hydrogenate the thin film layer 12 through its catalytic action. In this case, however, the deterioration of the catalyst layer 13 and the thin film layer 12 can be prevented more reliably, since the second protective film with such thickness can sufficiently reduce the amount of water, oxygen, and/or the like penetrating from the atmosphere into the catalyst layer 13 and the thin film layer 12. Hence, the thickness of the second protective film 15 may be determined appropriately, on the basis of a trade-off between hydrogen-gas detection time and life required on the hydrogen sensor.

Next, referring to FIG. 3, a modification of the hydrogen sensor according to the present invention will be described, where the constituents similar in function to those of the hydrogen sensor 10a will be assigned the same reference signs, and the description of such constituents will be omitted.

The hydrogen sensor 10b shown in FIG. 3 includes a substrate 11 of a material hardly absorbing water, oxygen and/or the like from the atmosphere, such as metal or glass. In such hydrogen sensor 10b, water, oxygen and/or the like scarcely penetrates from the substrate 11 into the thin film layer 12. Hence, even without the first protective film 14, the second protective film 15 can prevent water, oxygen and/or the like from penetrating from the atmosphere into the catalyst layer 13 and the thin film layer 12, thereby preventing deterioration of the catalyst layer 13 and the thin film layer 12. Consequently, the hydrogen-gas detection function of the hydrogen sensor 10b can be maintained satisfactorily for a long period of time.

The hydrogen sensor according to the present invention is not limited to the embodiments described above, but can be modified in various ways without departing from the spirit of the present invention.

## Claims

1. A hydrogen sensor comprising:
a substrate;
a thin film layer formed over the substrate; and
a catalyst layer formed on a surface of the thin film layer for causing the thin film layer to be hydrogenated by hydrogen gas in an atmosphere, thereby causing a change in optical reflectance of the thin film layer;
wherein a protective film is formed at least either between the substrate and the thin film layer or on a surface of the catalyst layer.

2. The hydrogen sensor according to claim 1,
wherein said thin film layer is a magnesium-nickel alloy thin film layer or a magnesium thin film layer.

3. The hydrogen sensor according to claim 2,
wherein said catalyst layer is formed of palladium or platinum.

4. The hydrogen sensor according to claim 3,
wherein said catalyst layer has a thickness between 1nm and 100nm.

5. The hydrogen sensor according to claim 1,
wherein said protective film is formed of a silicon compound, a fluorine compound, a fat or an oil.

6. The hydrogen sensor according to claim 5,
wherein said protective film has a thickness between 5nm and 200nm.

## Amended claims

### Amended claims under Art. 19.1 PCT

**1.** (Amended) A hydrogen sensor comprising:
a substrate;
a thin film layer formed over the substrate; and
a catalyst layer formed on a surface of the thin film layer for causing the thin film layer to be hydrogenated by hydrogen gas in an atmosphere, thereby causing a change in optical reflectance of the thin film layer;
wherein a protective film is formed at least between the substrate and the thin film layer.

**2.** The hydrogen sensor according to claim 1,
wherein said thin film layer is a magnesium-nickel alloy thin film layer or a magnesium thin film layer.

**3.** The hydrogen sensor according to claim 2,
wherein said catalyst layer is formed of palladium or platinum.

**4.** The hydrogen sensor according to claim 3,
wherein said catalyst layer has a thickness between 1nm and 100nm.

**5.** The hydrogen sensor according to claim 1,
wherein said protective film is formed of a silicon compound, a fluorine compound, a fat or an oil.

**6.** The hydrogen sensor according to claim 5,
wherein said protective film has a thickness between 5nm and 200nm.

Statement under Art. 19.1 PCT
**1.** has been amended to make clear that, in a hydrogen sensor comprising a substrate, a thin film layer formed over the substrate, and a catalyst layer formed on a surface of the thin film layer for causing the thin film layer to be hydrogenated by hydrogen gas in an atmosphere, thereby causing a change in optical reflectance of the thin film layer, a protective film is formed at least between the substrate and the thin film layer.
 Cited documents 1 to 3 each disclose a hydrogen sensor including a protective film formed on a surface of a catalyst layer provided in a form of a thin film. Unlike the present invention, the hydrogen sensors disclosed in these cited documents do not have a protective film between the substrate and the layer corresponding to the thin film layer of the present invention.
 With a protective film formed between the substrate and the thin film layer, the present invention can prevent the water, oxygen and/or the like absorbed into the substrate from penetrating into the protective layer.
